# EUROPEAN PATENT SPECIFICATION

(11) **EP 4 460 250 B1**
(45) Date of publication and mention of the grant of the patent: **17.12.2025**
(21) Application number: 23712807.9
(22) Date of filing: 09.01.2023
(51) Int. Cl.: A61B 17/92, A61F 2/46

(54) **IMPROVEMENTS IN AND RELATING TO DEVICES FOR SURGICAL INSTRUMENT EXTRACTION**
VERBESSERUNGEN AN UND IM ZUSAMMENHANG MIT VORRICHTUNGEN ZUR EXTRAKTION CHIRURGISCHER INSTRUMENTE
AMÉLIORATIONS APPORTÉES ET SE RAPPORTANT À DES DISPOSITIFS D'EXTRACTION D'INSTRUMENT CHIRURGICAL

(30) Priority: 07.01.2022 GB 202200194
(43) Date of publication of application: 13.11.2024
(73) Proprietor: DePuy Ireland Unlimited Company, County Cork (IE)
(72) Inventor: ATKIN, Jamie, Leeds Yorkshire LS11 8DT (GB); DUTTON, Graeme, Leeds Yorkshire LS11 8DT (GB); KARUNARATNE, Dinalie, Leeds Yorkshire LS11 8DT (GB); ROBINSON, Stephen, Leeds Yorkshire LS11 8DT (GB)
(74) Representative: Murgitroyd & Company
(86) International application number: PCT/EP2023/050291
(87) International publication number: WO 2023/131699

(56) References cited:
- FR-A1- 2 646 768
- US-A- 4 399 813
- US-A1- 2009 112 209
- US-A1- 2013 204 265
- US-A1- 2021 353 430

## Description

### TECHNICAL FIELD

The present disclosure relates generally to orthopaedic devices, kits including such devices and methods of using such devices and more particularly to orthopaedic surgical instrument extraction devices, kits including such devices and methods of using such devices.

### BACKGROUND

Joint arthroplasty is a well-known surgical procedure by which a diseased and/or damaged natural joint is replaced by a prosthetic joint. Examples include knee prosthesis and hip implants. Surgical instruments are used to prepare the bone for the receipt of further surgical instruments and/or implants and a reliable extraction device for those surgical instruments is desirable. Each of US2013/204265 and US2021/353430 discloses a surgical device for extraction of a prosthetic component from a patient.

### SUMMARY

According to a first aspect of the invention there is provided a surgical instrument extraction device, the extraction device comprising:
a body;
a first mounting location provided at the proximal end of the body;
a second mounting location provided at the distal end of the body;
wherein the second mounting location includes an engagement element for the surgical instrument to be extracted in use, the engagement element having a first extent between a first pair of points on opposite sides of the engagement element and having a second extent between a second different pair of points on opposite sides of the engagement element, wherein the first extent is greater than the second extent, the engagement element further including one or more non-planar surfaces.

The engagement element may include one or more non-planar surface sections, for instance curved surface sections and/or part-spherical surface sections. The engagement element may provide one or more engagement surface sections. The engagement element may provide one or more non-planar engagement surface sections, for instance curved surface sections and/or part-spherical surface sections. One or more or all, of the non-planar surface sections may provide engagement surface sections. One or more or all, of the non-planar surface sections may be curved surface sections and/or part-spherical surface sections.

One or more non-planar engagement surface sections may be provided adjacent the junction between the second mounting location and the body, for instance adjacent the junction between the engagement element and the elongate portion.

One or more non-planar engagement surface sections may be provided distal the junction between the second mounting location and the body, for instance opposite the junction between the engagement element and the elongate portion. One or more non-planar engagement surface sections may be provided opposite the junction between the second mounting location and the body, for instance opposite the junction between the engagement element and the elongate portion.

The engagement element comprises one or more surface sections which lie on a common sphere. The engagement element may comprise one surface sections which lies on a common sphere.

The engagement element may comprise one or more other surface sections. The engagement element may comprise one or more planar other surface sections. The engagement element may comprise two planar other surface sections. The engagement element may comprise at least two planar other surfaces which are coplanar with one another and/or extend parallel to the longitudinal axis of the extraction device.

The engagement element may have the profile of a sphere with one or more spherical caps removed. The plane defining a first spherical cap removed may be the plane forming the junction between the engagement element and the elongate portion. The plane defining a second spherical cap removed may be a first planar other surface, for instance which is coplanar with a second planar other surface and/or extends parallel to the longitudinal axis of the extraction device. The plane defining a third spherical cap removed may be a second planar other surface, for instance which is coplanar with a first planar other surface and/or extends parallel to the longitudinal axis of the extraction device. The plane defining the first spherical cap removed may touch and/or intersect the plan defining the second spherical cap removed. The plane defining the first spherical cap removed may touch and/or intersect the plane defining the third spherical cap removed.

The engagement element may have a first extent, for instance between a first pair of points on opposite sides of the engagement element. The engagement may have a second extent, for instance between a second different pair of points on opposite sides of the engagement element.

The first extent may be greater than the second extent. The first extent may be at least 10% greater than the second extent. The first extent may be at least 20%, or at least 25% or at least 30% greater than the second extent.

The first extent may be between a first pair of points on two different non-planar surfaces, for instance on two non-planar surfaces which are in opposition to one another and/or on two non-planar surfaces which are on opposite sides of the engagement element to one another.

The second extent may be between a second pair of points on two different planar surface sections, for instance on two planar surfaces which are coplanar with one another and/or extend parallel to the longitudinal axis of the extraction device.

The first extent and/or the second extent may be considered along an axis and/or in a plane, perpendicular to the longitudinal axis of the extraction device and/or of the body portion. The first extent may be considered along an axis perpendicular to a point on a first non-planar surface and/or perpendicular to a point on a second non-planar surface. The second extent may be considered along an axis and/or in a plane, perpendicular to the plane of a first planar surface section and/or perpendicular to the plane of a second surface section.

References in this document to perpendicular to, may in one or more or all of those instances include perpendicular to +/- 10°, or +/- 8°, or +/- 5°, or +/- 3°.

The body may include a body portion. The body may include an elongate portion. The body may include a transition portion. The transition portion may connect the body portion to the elongate portion.

The first mounting location may be provided by the body portion, particularly in the proximal end of the body portion. The second mounting location may be provided on the elongate portion, particularly on the distal end of the elongate portion.

The first mounting location may be adapted to connect the extraction device to the distal end of a tool

The first mounting location may include a bore. The bore may be provided in the body, for instance in a body portion. The first mounting location may provide a screw thread. The first mounting location may provide a bore with a female screw thread provided within it.

The first mounting location may be adapted to connect, for instance receive, a distal end of a tool. The first mounting location may be adapted to engage with a screw thread on the tool, for instance a male screw thread.

The first mounting location may have a tool engaged state and a tool disengaged state. The first mounting location may transition from the tool engaged state to the tool disengaged state and/or from the tool disengaged state to the tool engaged state by rotation of the first mounting location, for instance rotation of the extraction device.

The extraction device may have a longitudinal axis, for instance adapted to align with a longitudinal axis of the tool.

The body may include a first cross-sectional area part and a second cross-sectional area part, which is closer to the distal end of the extraction device. The body portion may include the first cross-sectional area part. The elongate portion may include the second cross-sectional area part. The body may include a third cross-sectional area part. The transition part may include the third cross-sectional are part.

The first cross-sectional area part may include the first mounting location.

The first cross-sectional area part may have a greater cross-sectional area than the second cross-sectional area part. The first cross-sectional area part may have a greater cross-sectional area that the third cross-sectional area part.

The first cross-sectional area part may have a circular cross-sectional area. The first cross-sectional area part may have a partially circular cross-sectional area. The first cross-sectional area part may include two or more linear parts to its perimeter. Two or more linear parts may be provided in opposition to one another and/or parallel to one another.

The second cross-sectional area part may lead to the second mounting location, particularly at the distal end thereof.

The second cross-sectional area part may have a lesser cross-sectional area than the third cross-sectional area part.

The second cross-sectional area part may have a circular cross-sectional area.

The third cross-sectional area part may have a circular cross-sectional area.

The body, for instance the body portion, may be or include a cylindrical portion. The body portion may include one or more planar surfaces. One or more pairs of planar surfaces may be provided, for instance with the planar surfaces of the pair in opposition to one another. The planar surfaces may be provided parallel to the longitudinal axis of the extraction device.

The body, for instance the transition portion, may be or include a conical portion. The transition portion may transition from the shape and configuration of the body portion to the shape and configuration of the elongate portion.

The body, for instance the elongate portion, may be or include a cylindrical portion. The body, for instance the elongate portion, may be or include a neck portion.

The extraction device may have a tool disengaged state. The extraction device may have a tool engaged state. The extraction device may have an instrument disengaged state. The extraction device may have an instrument contacting state. The extraction device may have an instrument engaged state. The extraction device may have an alternative orientation state. The extraction device may have an instrument withdrawing state.

The tool disengaged state may provide the extraction device spaced from the tool. The tool engaged state may provide the extraction device mounted on the tool. The tool engaged state may provide restraint against movement of the extraction device away from the tool axially.

The extraction device may pass through a first transition stage between the tool disengaged state and the tool engaged state. The extraction device and the tool may be rotated relative to one another and/or be advanced axially toward one another in the first transition stage.

The instrument disengaged state may provide the extraction device mounted on the tool and/or spaced from the instrument.

The instrument contacting state may provide a part of the extraction device, for instance the second mounting location in contact with a part of the instrument. The second mounting location may be partially or fully inserted into the instrument. The instrument contacting state may provide no restraint against movement of the extraction device and/or instrument away from one another axially. The instrument contacting state may provide no restraint against rotation of the extraction device relative to the instrument.

The extraction device may pass through a second transition stage between the instrument disengaged state and the instrument contacting state. The extraction device and the instrument may be advanced axially toward one another in the second transition stage. The extraction device and the instrument may not be rotated relative to one another in the second transition stage.

In the second transition stage, the engagement element may provide a first face, such as a first planar face as an anterior facing face, and/or the engagement element may provide a second face, such as a second planar face as a posterior facing face. In the second transition stage, the maximum anterior-posterior dimension of the second mounting location may be less than the minimum anterior-posterior dimension of an instrument mounting location provided by the instrument. In the second transition stage, the second mounting location may be able to enter the instrument mounting location provided by the instrument.

In other orientations of the second mounting location, for instance in the tool disengaged state and/or the tool engaged state and/or the instrument disengaged state and/or the instrument engaged state and/or the one or more alternative orientation states, the second mounting location may be unable to enter the instrument mounting location and/or unable to leave the instrument mounting location. In one or more other orientations of the second mounting location, the engagement element may provide a first face, such as a first planar face as a non-anterior facing face, and/or the engagement element may provide a second face, such as a second planar face as a non-posterior facing face. In one or more or all of the other orientations, the maximum anterior-posterior dimension of the second mounting location may be greater than the minimum anterior-posterior dimension of an instrument mounting location provided by the instrument. In one or more or all of the other orientations, the second mounting location may be unable to enter the instrument mounting location provided by the instrument.

One or more of all of the other orientations may be transitions to a second transition stage by rotation of the extraction device, for instance to provide an alignment to allow the second mounting location to pass into the instrument.

The instrument engaging state may provide a part of the extraction device, for instance the second mounting location engaged with a part of the instrument. The second mounting location may be fully inserted into the instrument. The instrument engaging state may provide restraint against movement of the extraction device and/or instrument away from one another axially. The instrument engaging state may provide no restraint against rotation of the extraction device relative to the instrument.

The extraction device may pass through a third transition stage between the instrument contacting state and the instrument engaging state. The extraction device and the instrument may be rotated relative to one another in the third transition stage. The rotation may be 90° +/- 40°. The rotation may be 90° +/- 30°. The rotation may be 90° +/-20°. The rotation may be 90° +/- 10°.

The extraction device may have one or more alternative orientation states, particularly whilst in the instrument engaging state. The one or more alternative orientation states may be a different angle[s] of the longitudinal axis of the extraction device relative to the direction of movement during the second transition stage and/or relative to the longitudinal axis of the instrument and/or relative to an axis or plane parallel to the longitudinal axis of the instrument.

The extraction device may have a series of alternative orientation states over a range of orientation states spanning more than 10° of arc, for instance more than 20° of arc, such as more that 30° of arc or even up to 45° of arc. The extraction device may have a series of alternative orientation states over a range of orientation states spanning less than 60° of arc, for instance less than 50° of arc. The extraction device may have a series of alternative orientation states over a range of 0° to 10° in a medial direction relative to the longitudinal axis of the instrument and/or relative to an axis or plane parallel to the longitudinal axis of the instrument. The extraction device may have a series of alternative orientation states over a range of 20° to 50°, for instance 30° to 40°, in a lateral direction relative to the longitudinal axis of the instrument and/or relative to an axis or plane parallel to the longitudinal axis of the instrument. The one or more alternative orientation states may provide restraint against movement of the extraction device and/or instrument away from one another axially. The one or more alternative orientation states may provide no restraint against rotation of the extraction device relative to the instrument.

The extraction device may transition from one alternative orientation state to another alternative orientation state by rotating the extraction device relative to the instrument, for instance in the medial direction or lateral direction.

The instrument withdrawing state may provide an axial load on the extraction device away from the instrument. The axial load may be applied via the tool. The axial load may be constant or may vary, for instance through the action of a slap hammer to the tool. The instrument withdrawing state may provide relative movement of the instrument and the patient.

The body, for instance the body portion, may be provided with one or more visible indicators of the orientation of the second mounting location. The visible indicators may be one or more planar surfaces on the body portion which are in the same plane or a parallel plane to a planar surface of the second mounting location. The visible indicators may be outside of the instrument and/or visible in all states and stages.

In an alternative embodiment, the engagement element has a non-spherical profile. The engagement element may be provided with a cylindrical part. The cylindrical part may have an axis inclined relative to the axis of the extraction device, for instance with an axis perpendicular to the axis of the extraction device +/- 5°. The axis or a parallel axis, may define the first extent. The second extent may be defined along an axis perpendicular to the axis defining the first extent.

A right cylindrical part may be provided.

In the contacting state, the axis of the cylindrical part may extent laterally-medially. In the engaging state the axis of the cylindrical part may extend anteriorly-posteriorly.

In a further alternative embodiment, the engagement element has a non-spherical profile. The engagement element may be provided with a rectangular part. The rectangular part may have a long axis inclined relative to the axis of the extraction device, for instance with an axis perpendicular to the axis of the extraction device +/- 5°. The axis or a parallel axis, may define the first extent. The second extent may be defined along an axis perpendicular to the axis defining the first extent.

In the contacting state, the long axis of the rectangular part may extent laterally-medially. In the engaging state the long axis of the rectangular part may extend anteriorly-posteriorly.

According to a second aspect of the invention there is provided a kit comprising:
a surgical instrument extraction device, the extraction device comprising:
   a body;
   a first mounting location provided at the proximal end of the body;
   a second mounting location provided at the distal end of the body;
wherein the second mounting location includes an engagement element for the surgical instrument to be extracted in use, the engagement element having a first extent between a first pair of points on opposite sides of the engagement element and having a second extent between a second different pair of points on opposite sides of the engagement element, wherein the first extent is greater than the second extent, the engagement element further including one or more non-planar surfaces which comprise one or more surface sections which lie on a common sphere,
the kit further comprising:
   a tool for attachment to the first mounting location of the surgical instrument extraction device.

The tool may further include a handle, for instance at the proximal end of the tool. The tool may further include one or more abutment surfaces, for instance one or more radially extending elements or flanges. The one or more abutment surfaces may be adapted for impact by a further tool, such as a slap hammer.

The extraction device mounting location may be a complimentary mounting location to the first mounting location of the extraction device. The extraction device mounting location may include a stem. The stem may extend from the tool. The extraction device mounting location may provide a screw thread. The first extraction device mounting location may provide a stem with a male screw thread provided on its outside.

The extraction device mounting location may be adapted to connect, for instance receive, a proximal end of the extraction device. The extraction device mounting location may be adapted to engage with a screw thread on the extraction device, for instance a female screw thread.

The extraction device mounting location may have an extraction device engaged state and an extraction device disengaged state. The extraction device mounting location may transition from the extraction device engaged state to the extraction device disengaged state and/or from the extraction device disengaged state to the extraction device engaged state by rotation of the extraction device mounting location, for instance rotation of the tool. The tool may be adapted to perform one or more further roles, besides engagement with the extraction device. The tool could also serve as a stem extraction shaft for use in extracting stems.

According to a third aspect of the disclosure there is provided a surgical instrument, the surgical instrument including a tapered body extending distally from a first end to a second end, one or more sections of the tapered body being provided with a plurality of cutting teeth, a superior surface of the tapered body being provided with an instrument mounting location, wherein the mounting location is provided within an opening and wherein the opening has a first extent between a first pair of locations on opposite sides of the opening or slot and has a second extent between a second different pair of locations on opposite sides of the opening or slot, wherein the first extent is greater than the second extent.

The instrument may be a broach.

The broach may have a superior portion. The superior portion may be provided with a series of teeth and/or recesses, particularly teeth alternating with recesses. The broach may have a calcar face. The calcar face may be provided with a projection, such as a spigot, for instance for receiving a trial or a part thereof.

The calcar surface may be provided with an opening. The opening may be a slot. The slot may be a closed end slot. The slot may be an open end slot, for instance at the medial end. The opening may be an aperture. The opening may be an aperture which is connected to a second aperture in an inferior face of the broach. The opening may be partially defined by one or more perimeter wall[s]. The opening may be connected to a chamber.

The chamber may be recessed into the instrument, for instance inferior to the perimeter wall[s] defining the opening. The chamber may be partially defined by one or more side walls and/or a base wall. The chamber may be further defined by a roof wall through which the opening extends. The anterior extent of the chamber may be greater than the anterior extent of the opening. The posterior extent of the chamber may be greater than the posterior extent of the opening. The lateral extent of the chamber may be greater than the lateral extent of the opening. The medial extent of the chamber may be greater than the medial extent of the opening.

The chamber may undercut the opening in one or more directions.

The opening and/or slot and/or the chamber may have a width, considered anteriorly-posteriorly, which is less than the length of the slot, considered medially-laterally. The opening and/or slot and/or the chamber may have a second extent, considered anteriorly-posteriorly, which is less than the first extent, considered medially-laterally.

The first extent of the opening and/or slot and/or the chamber may be greater than the first extent of the extraction device. The second extent of the opening and/or slot and/or the chamber may be less than the first extent of the extraction device. The second extent of the opening and/or slot and/or the chamber may be greater than the second extent of the extraction device.

The chamber may provide one or more contact surfaces for one or more surfaces of the second mounting location of the extraction device. The one or more contact surfaces may provide articulation surfaces, for instance to allow rotation of the extraction device relative to the instrument. The chamber may provide one or more non-planar surface sections, for instance complimentary to one or more non-planar surface sections of the extraction device. The base of the chamber may provide one or more non-planar surface sections, for instance complimentary to one or more non-planar surface sections of the extraction device. The roof of the chamber may provide one or more non-planar surface sections, for instance complimentary to one or more non-planar surface sections of the extraction device. The end, for instance the lateral end of the chamber may provide one or more non-planar surface sections, for instance complimentary to one or more non-planar surface sections of the extraction device. The side walls of the chamber may provide one or more non-planar surface sections, for instance complimentary to one or more non-planar surface sections of the extraction device.

The opening and/or slot may limit the range of variations possible in the axial alignment of the tool and/or extraction device, for instance by abutment.

The instrument may be provided with one or more teeth and/or recesses, for instance on the anterior and posterior faces. The instrument may be provide with tooth and recess free space on or near the lateral shoulder face, for instance provided with alpha/numerical markings

The third aspect may include any of the features, possibilities or options set out elsewhere in this document, including in the other aspects.

According to a fourth aspect of the disclosure there is provided a method extracting a surgical instrument from a patient, the method comprising:
1) providing a surgical instrument extraction device, the extraction device comprising:
   a body;
   a first mounting location provided at the proximal end of the body;
   a second mounting location provided at the distal end of the body;
   wherein the second mounting location includes an engagement element for the surgical instrument to be extracted in use, the engagement element having a first extent between a first pair of points on opposite sides of the engagement element and having a second extent between a second different pair of points on opposite sides of the engagement element, wherein the first extent is greater than the second extent, the engagement element further including one or more non-planar surfaces.
2) inserting the engagement element of the extraction device into an opening in the surgical instrument to provide an instrument contacting state;
3) rotating the extraction device relative to the surgical instrument to give an an instrument engaged state;
4) increasing the separation between the extraction device and the patient to withdraw the surgical instrument from the patient.

The method may include a tool disengaged state for the extraction device. The method may include a tool engaged state. The method include an instrument disengaged state. The method may include an instrument contacting state. The method may include an instrument engaged state. The method may include an alternative orientation state. The method may include an instrument withdrawing state.

The tool disengaged state may provide the extraction device spaced from the tool.

The tool engaged state may provide the extraction device mounted on the tool. The tool engaged state may provide restraint against movement of the extraction device away from the tool axially. The method may further include, connecting a tool to the extraction device to provide the tool engaged state. The tool may be connected to the extraction device before step 2) is commenced. The tool may be connected to the extraction device during step 3 and/or step 4.

The method may include the extraction device passing through a first transition stage between the tool disengaged state and the tool engaged state. The method may include rotating the extraction device and the tool relative to one another and/or being advanced axially toward one another during the first transition stage.

The instrument disengaged state may provide the extraction device mounted on the tool and/or spaced from the instrument.

The instrument contacting state may provide a part of the extraction device, for instance the second mounting location in contact with a part of the instrument. The second mounting location may be partially or fully inserted into the instrument. The instrument contacting state may provide no restraint against movement of the extraction device and/or instrument away from one another axially. The instrument contacting state may provide no restraint against rotation of the extraction device relative to the instrument.

The method may include the extraction device passing through a second transition stage between the instrument disengaged state and the instrument contacting state. The method may include the extraction device and/or the instrument being advanced axially toward one another during the second transition stage. The extraction device and the instrument may not be rotated relative to one another during the second transition stage.

During the second transition stage, the engagement element may provide a first face, such as a first planar face as an anterior facing face, and/or the engagement element may provide a second face, such as a second planar face as a posterior facing face. During the second transition stage, the maximum anterior-posterior dimension of the second mounting location may be less than the minimum anterior-posterior dimension of an instrument mounting location provided by the instrument. During the second transition stage, the second mounting location may be able to enter the instrument mounting location provided by the instrument.

The method may provide that in other orientations of the second mounting location, for instance in the tool disengaged state and/or the tool engaged state and/or the instrument disengaged state and/or the instrument engaged state and/or the one or more alternative orientation states, that the second mounting location may be unable to enter the instrument mounting location and/or unable to leave the instrument mounting location. The method may provide that in one or more other orientations of the second mounting location, the engagement element may provide a first face, such as a first planar face as a non-anterior facing face, and/or the engagement element may provide a second face, such as a second planar face as a non-posterior facing face. The method may provide that in one or more or all of the other orientations, the maximum anterior-posterior dimension of the second mounting location may be greater than the minimum anterior-posterior dimension of an instrument mounting location provided by the instrument. The method may provide that in one or more or all of the other orientations, the second mounting location may be unable to enter the instrument mounting location provided by the instrument.

The method may provide that one or more of all of the other orientations may be transitions to a second transition stage by rotation of the extraction device, for instance to provide an alignment to allow the second mounting location to pass into the instrument.

The method may include the instrument engaging state providing a part of the extraction device, for instance the second mounting location engaging with a part of the instrument. The second mounting location may be fully inserted into the instrument. The method may include the instrument engaging state providing restraint against movement of the extraction device and/or instrument away from one another axially. The instrument engaging state may provide no restraint against rotation of the extraction device relative to the instrument.

The method may include the extraction device passing through a third transition stage between the instrument contacting state and the instrument engaging state. The extraction device and the instrument may be rotated relative to one another during the third transition stage. The rotation may be 90° +/- 40°. The rotation may be 90° +/- 30°. The rotation may be 90° +/- 20°. The rotation may be 90° +/- 10°.

The method may include the extraction device having one or more alternative orientation states, particularly whilst in the instrument engaging state. The one or more alternative orientation states may be a different angle[s] of the longitudinal axis of the extraction device relative to the direction of movement during the second transition stage and/or relative to the longitudinal axis of the instrument and/or relative to an axis or plane parallel to the longitudinal axis of the instrument.

The method may include the extraction device having a series of alternative orientation states over a range of orientation states spanning more than 10° of arc, for instance more than 20° of arc, such as more that 30° of arc or even up to 45° of arc. The method may include the extraction device having a series of alternative orientation states over a range of orientation states spanning less than 60° of arc, for instance less than 50° of arc. The method may include the extraction device having a series of alternative orientation states over a range of 0° to 10° in a medial direction relative to the longitudinal axis of the instrument and/or relative to an axis or plane parallel to the longitudinal axis of the instrument. The method may include the extraction device having a series of alternative orientation states over a range of 20° to 50°, for instance 30° to 40°, in a lateral direction relative to the longitudinal axis of the instrument and/or relative to an axis or plane parallel to the longitudinal axis of the instrument. The method may include the one or more alternative orientation states providing restraint against movement of the extraction device and/or instrument away from one another axially. The method may include the one or more alternative orientation states providing no restraint against rotation of the extraction device relative to the instrument.

The method may include the extraction device transitioning from one alternative orientation state to another alternative orientation state by rotating the extraction device relative to the instrument, for instance in the medial direction or lateral direction.

The method may include the instrument withdrawing state in a plurality of different alternative orientation states. The method may include the instrument withdrawing state providing an axial load on the extraction device away from the instrument. The axial load may be applied via the tool. The axial load may be constant or may vary, for instance through the action of a slap hammer to the tool. The instrument withdrawing state may provide relative movement of the instrument and the patient.

The fourth aspect may include any of the features, possibilities or options set out elsewhere in this document, including in the other aspects.

### BRIEF DESCRIPTION OF THE DRAWINGS

Various embodiments of the disclosure will now be described, by way of example only, and with reference to the accompanying figures, in which:
Figure 1 is a perspective side view of a first embodiment of a broach extraction device;
Figure 2a is a perspective view of an embodiment of a broach extraction device approaching a broach, a disengaged state;
Figure 2b is a perspective view of the combination of Figure 2a, with the broach extraction device in an inserted state relative to the broach;
Figure 2c is a perspective view of the combination of Figure 2a and 2b, with the broach extraction device in a locked state relative to the broach;
Figure 3a is a cross-sectional side view of an embodiment of a broach extraction device in a locked state relative to a broach;
Figure 3b is a detailed view of the Figure 3a combination showing a number of possible orientations/inclinations for the broach extraction device relative to the broach;
Figure 4a is anterior perspective view of a further embodiment of a broach;
Figure 4b is a lateral superior perspective view of the broach according to the Figure 4a embodiment;
Figure 5a is anterior view of a still further embodiment of a broach and broach extraction device in the disengaged state;
Figure 5b is the view of Figure 5a with the broach and broach extraction device in the inserted state;
Figure 5c is the view of Figure 5a and 5b with the broach and broach extraction device in the locked state;
Figure 5d shows an alternative inclination, in the locked state, for the broach and broach extraction device of Figure 5c;
Figure 6 shows another embodiment of a broach and broach extraction element;
Figure 7a shows another embodiment of a broach and a broach extraction element in the disengaged state; and
Figure 7b shows the broach and broach extraction element of Figure 7a in the locked state.

### DETAILED DESCRIPTION OF THE DRAWINGS

While the concepts of the present disclosure are susceptible to various modifications and alternative forms, specific embodiments thereof have been shown by way of example in the drawings and will be described herein in detail. It should be understood, however, that there is no intent to limit the concepts of the present disclosure to the particular forms disclosed, but on the contrary, the intention is to cover all modifications, equivalents, and alternatives consistent with the present disclosure and the appended claims.

Terms representing anatomical references, such as anterior, posterior, medial, lateral, superior, inferior, et cetera, may be used throughout the specification in reference to the orthopaedic implants or prostheses and surgical instruments described herein as well as in reference to the patient's natural anatomy. Such terms have well-understood meanings in both the study of anatomy and the field of orthopaedics. Use of such anatomical reference terms in the written description and claims is intended to be consistent with their well-understood meanings unless noted otherwise.

In hip arthroplasty, a femoral component of the prosthesis must be successfully introduced into the prepared proximal end of the femur. Press-fit implantation is frequently used and this requires a close fit between the implant and the internal surface of the femur. The close fit is needed to promote bone ingrowth and give the needed load transfer, without allowing motion at the bone/implant interface.

To achieve this, controlled preparation of the medullary canal is provided using increasing size reamers and broaches to prepare the femur. The first broach used is typically two or three sizes below that of the pre-operatively templated size for the implant. The broaches are used sequentially; each being inserted to increase the canal dimensions and then removed. The final size of broach is typically used with the trial prosthesis attached to allow for proper assessment of the configuration provided. Once the trial has been completed, the final size of preparatory broach is removed and the final implantation can be performed using the correct stem size relative to that final broach.

In some instances, removal of one or more of the broaches with the associated broach impactor handles can be difficult, for example in the case of the broach spigot connection feature becoming damaged or fractured, and so a dedicated broach extractor is provided in existing instrument sets to assist with this removal. It is beneficial to be able to remove the broach successfully, without any further bone or tissue removal.

A broach becoming stuck in the femur can increase the time taken to complete the surgery and/or lead to complications in the surgical procedures needed. Hence, effective removal of the broach is needed. In addition, the single axial orientation in the existing approach and the position of the engagement with the broach can be sub-optimal with some patient anatomies.

The present disclosure seeks to provide a lower space requirement for engagement between the broach extractor and the broach. The present disclosure seeks to avoid the need for and take up the space of a further device in the instrument set by providing a small attachment for existing devices. The present disclosure seeks to provide an engagement optimized for the size of broach in question, with minimum number of instruments required.

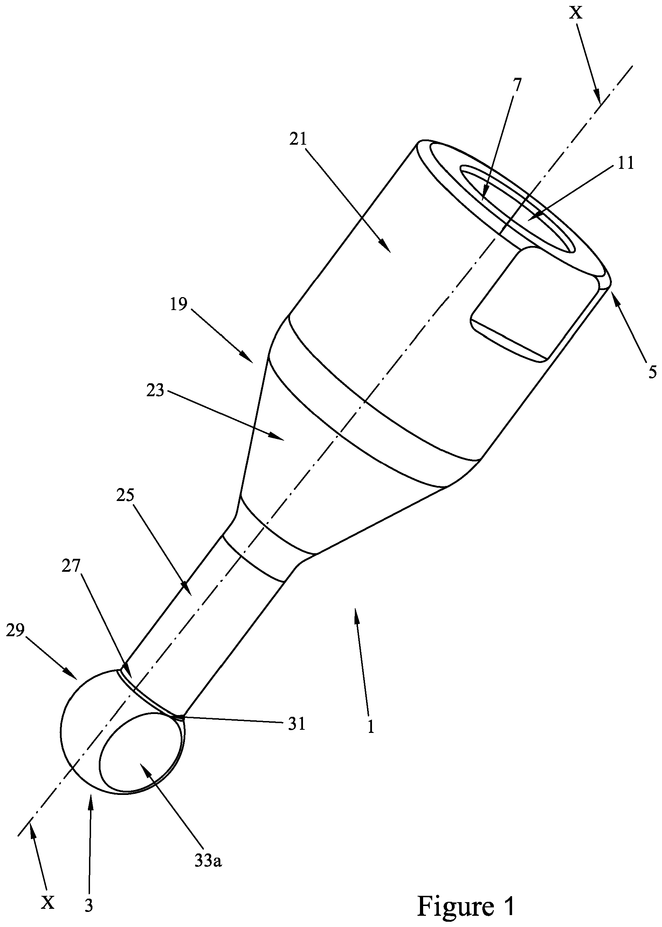

A first embodiment of broach extraction device 1 according to the disclosure is illustrated in Figure 1. The broach extraction device 1 is provided with a distal end 3 and a proximal end 5.

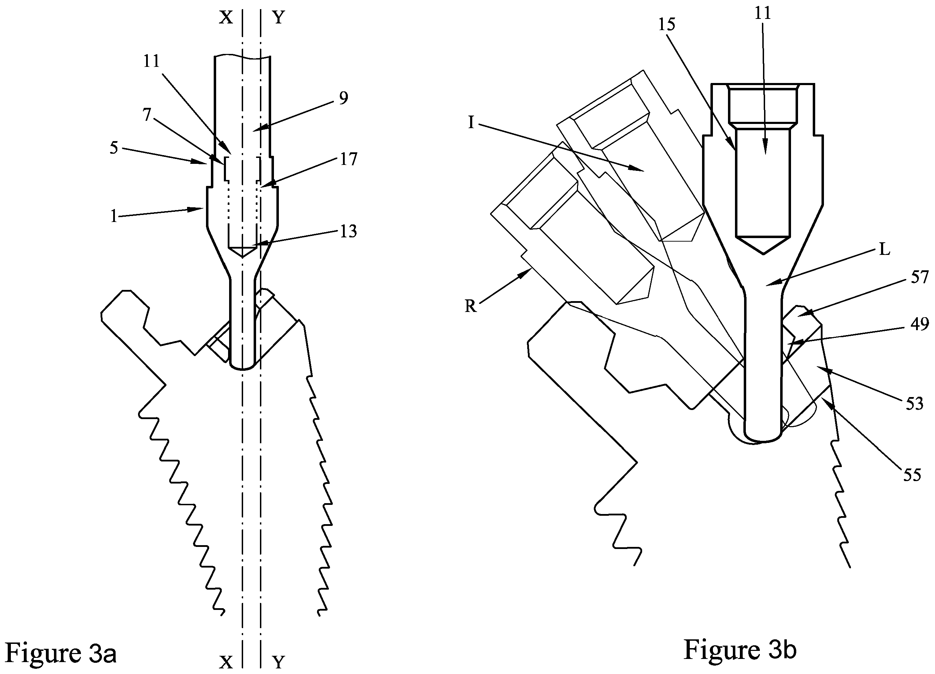

As seen in Figure 3a, the proximal end 5 provides a mounting location 7 for connecting the broach extraction device 1 to the distal end of a tool, for instance an extraction shaft 9. Various configurations for the mounting location 7 are possible, but each have a suitable configuration for engagement with the configuration of the distal end 13 of the tool, such as extraction shaft, 9, employed. In the illustrated configuration, the bore 11 in the proximal end 5 has a female screw thread provided within it.

The bore 11 receives the distal end 13 of the tool and promotes alignment between the female screw thread 15 and the male screw thread 17 provided on the distal end 13. Rotation of the broach extraction device 1 and the tool 9 relative to one another screws the two components together and provides a strong, single axial alignment engagement between them. In this configuration, the broach extraction device 1 is ready for use on the tool 9.

Because the broach extraction device 1 is a separate component from the tool 9, the broach extractor function can be provided by a surgical instrument which has other functions too. For instance, the tool 9 could also serve as a stem extraction shaft for use in extracting stems. The integral nature of the distal end 508 of the broach extractor 500 and its broach extraction in the prior art meant that this broach extractor 500 could only be used for that function.

Returning to Figure 1, the broach extraction device 1 has a body portion 19 which surrounds the bore 11. In the illustrated embodiment, the body portion 19 includes a right cylindrical part 21 and a conical part 23 which tapers down and provides the transition to a neck portion 25. The neck portion 25 is also a right cylinder, but has an appreciably lower diameter than the main part of the body portion 19. At the distal end 27 of the neck portion 25 is an engagement element 29.

In the embodiment of Figure 1, the engagement element 29 is a part sphere. The engagement element 29 is only a part sphere as the sphere is truncated by the plane 31 which defines the junction between the engagement element 29 and the neck portion 25. The engagement element 29 is only a part sphere as the sphere is truncated by opposing side flats 33a, 33b [not visible]. The flats 33a, 33b are aligned parallel to the longitudinal axis X-X of the broach extraction device 1. The distal end 3 is formed by the spherical part of the engagement element 29.

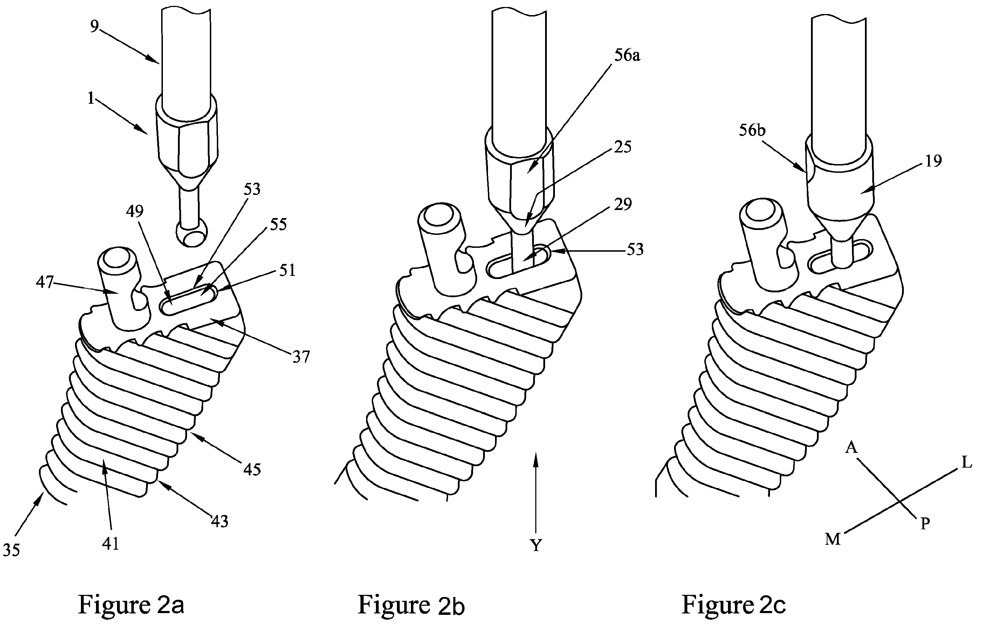

Figure 2a, 2b and 2c show the broach extraction device 1 in operation with respect to broach 35.

In Figure 2a, the broach extraction device 1 already mounted on the distal end 13 of the tool 9 by means of the threaded engagement described above. The broach extraction device 1 is advanced axially towards the calcar face 37 of the broach 35.

The broach 35 has a superior portion 41 visible in Figure 2a, which along with the rest of the stem [not shown] is provided with a series of teeth 43 alternating with recesses 45 to assist in the function of the broach 35. The calcar face 37 of the broach 35 extends medially-laterally, when inserted in the femur. The calcar face 37 is provided with a projection 47 which receives the trial neck segment [not shown] which in turn mounts the trial modular head [also not shown].

The calcar surface 37 is provided with a slot 49 which extends inferiorly into the broach 35. The slot 49 is defined by perimeter wall[s] 51 and opens into a chamber 53 partially defined by a base wall 55 [visible in Figure 2a]. The chamber 53 is describe in more detail below and has a greater width that the width of the slot 49. This forms an undercut. The slot 49 has a width, considered anteriorly-posteriorly A-P, which is less than the length of the slot, considered medially-laterally M-L.

As shown in Figure 2a, the flats 33a, 33b on the engagement element 29 face anteriorly-posteriorly A-P and so the reduced "width" of the engagement part 29 in that orientationally, due to the flats 33a, 33b, allows the engagement element 29 to enter the slot 49 and then enter the chamber 53 provided inferiorly to the slot 49.

If the flats 33a, 33b on the engagement element 29 are presented facing laterally-medially L-M, then the full "width" of the engagement element 29 in that orientationally, due to the full sphere of the engagement element 29 being presented, means that the engagement element 29 cannot enter the slot 49 and reach the chamber 53 provided inferiorly to the slot 49. Rotation of the extraction shaft 9 and the attached broach extraction device 1 is needed to provide correct alignment to allow the engagement element 29 to pass through the slot 49 and so transition from the disengaged state of Figure 2a to the inserted state of Figure 2b.

As shown in Figure 2b, the engagement element 29 is in the chamber 53 and a part of the neck portion 25 extends through the slot 49.

In the inserted state of Figure 2b, any attempt to move the tool 9 and the broach extraction device 1 in a proximal axial direction, arrow Y, will result in the transition back from the inserted state to the disengaged state as there is nothing to resist that movement and separation.

Referring to the inserted state of Figure 2b, if the tool 9 and the broach extraction device 1 are rotated, in either direction, but anti-clockwise in the example, then the engagement element 29 will also rotate within the chamber 53 and the neck portion 25 will rotate in the slot 49.

The body portion 19 of the broach extraction device 1 is provided with two flats 56a, 56b which are parallel to the flats 33a, 33b on the engagement element 29. Observation of the orientation of at least one of the flats 56a, 56b informs on the orientation of the flats 33a, 33b and hence of the engagement element 29 within the chamber 53. As shown, the flats 56a, 56b both extend the full length of the body portion 19, but the flats might only be provided on a sub-section of the body portion 19, for instance at the proximal end thereof [as seen in Figure 1].

Once rotated to a sufficient extent, such as 90° relative to the orientation shown in Figure 2a and 2b, then the flats 33a, 33b on the engagement element 29 are presented facing laterally-medially L-M. Hence, the full "width" of the engagement element 29, due to the full sphere part of the engagement element 29, is presented to the slot 49 in that orientation, and this means that the engagement element 29 cannot enter the slot 49 and so leave the chamber 53. This is the locked state shown in Figure 2c.

Once in the locked state, the broach extraction device 1 and the tool 9 can be considered to be in a retraction state and an axial force can be applied to the tool 9 to retract the broach 35 from the femur.

The part spherical engagement element 29 presents its spherical parts to the side wall sections 61 in this locked orientation and these side wall sections 61 have a corresponding profile to provide an articulating surface for the engagement element 29 and the chamber 53 relative to one another. This means that the broach engagement element 1 and tool 9 can be articulated or otherwise transitioned from the locked state and its retraction state to an axially aligned broach retraction state in which the axis X-X of the tool 9 and broach extraction device 1 is closely aligned or aligned with the broach axis Y-Y. This may be in the sense of the tool 9 and broach extraction device 1 have their axis X-X substantially parallel to, even if offset slightly from, the axis Y-Y of the broach, see Figure 3a. In the axially aligned broach retraction state, the axial force applied to the tool 9 is optimized in the orientation in which it is conveyed to the broach 35.

In the locked state of Figure 2c, any attempt to move the tool 9 and the broach extraction device 1 in a proximal axial direction, arrow Y, will result in the axial force being communicated to the broach 35, as the engagement element 29 is unable to leave the broach 35. In this manner, the tool 9 and the broach extraction device 1 can be used to remove the broach 35 from the femur [not shown].

Whilst the same axial alignment of the tool 9 and the broach extraction device 1 relative to the broach 35 is shown in each of Figures 2b and 2c, the embodiments of the disclosure allow for controlled variation in the axial alignment the tool 9 and the broach extraction device 1 relative to the broach 35 through the configuration of the slot 49, the engagement element 29 and the chamber 53. These configurations are best described with reference to Figures 3a and 3b.

Several features of the configuration of the slot 49, the engagement element 29 and the chamber 53 combine to allow, but still control, the variation in the axial alignment of the tool 9 and the broach extraction device 1.

Referring to Figure 3b, three illustrative axial alignments are shown. The intermediate illustration, I, shows an axial alignment generally consistent with the orientation used in Figures 3a, 2b and 2c to insert and engage the broach extraction device 1 with the broach 35. In this orientation, the broach extraction device 1 is clear of the projection 47 and clear of the lateral section 57 of the perimeter wall 51 of the slot 49.

The right-hand illustration, R, and the left-hand illustration, L, show close to the maximum medial deviation and close to the maximum lateral deviation respectively.

The left-hand illustration is confined from further inclination in a medial direction by the broach extraction device 1 abutting the projection 47 on the broach 35. Some further inclination in the medial direction might be possible if the engagement element 29 on the distal end of the broach extraction device 1 is moved laterally within the chamber 53 along the base wall 55.

The right-hand illustration is confined from further inclination in a lateral direction by the broach extraction device 1 abutting the lateral section 57 of the perimeter wall 51 around the slot 49. Slight medial movement of the engagement element 29 might be possible to give further inclination.

The range of inclinations possible is substantially greater than the single inclination intended with the prior art approach. This allows axial forces to be applied in different inclinations to help work the broach 35 free from the femur.

Returning to Figure 3b, the constraint on the inclination provided by the lateral section 57 is beneficial in preventing the tool 9 and/or broach extraction device 1 from contacting the soft tissue likely to be present medially of the broach 35 and the incision made to allow access to the femur.

The right-hand illustration, and Figure 3a position, is an orientation which is particularly useful for extracting the broach as the axial alignment of the tool 9 and the broach extraction device 1 is aligned with the axis of the broach 35 within the femur. This means that the axial force is better aligned to remove the broach 35 from the femur.

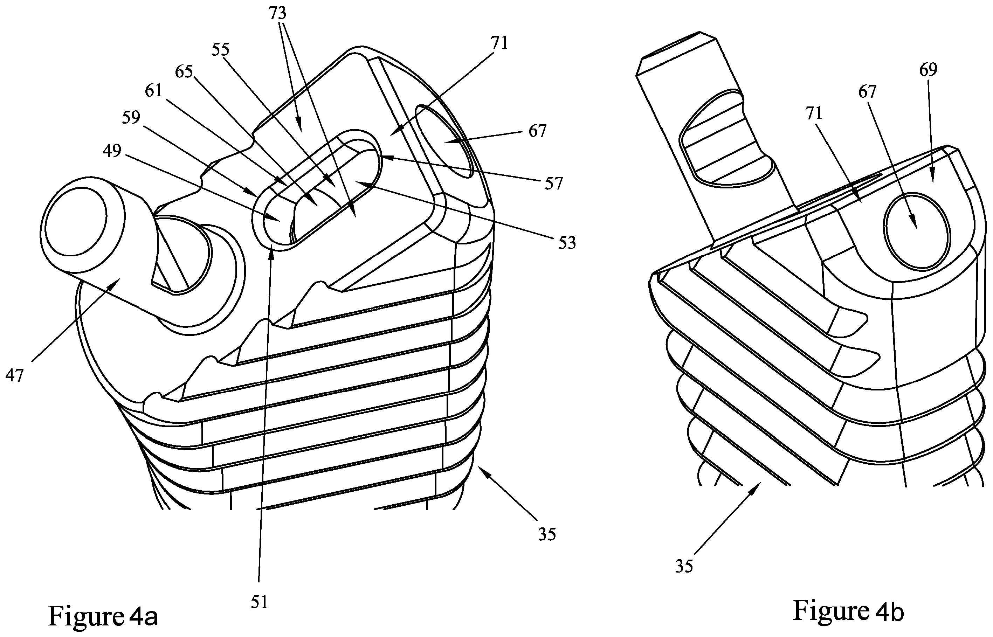

The configuration of the slot 49, perimeter wall 51, lateral section 57, chamber 53 and base wall 55 are clearly illustrated in Figure 4a. The slot 49 is provided with a chamfered edge section 59 which extends around the perimeter of the slot 49 and is part of the perimeter wall 51. The chamfered edge section 59 transitions into a side wall section 61 which forms the remainder of the perimeter wall 51 and defines part of the chamber 53. The chamber 53 is further defined by the base wall 55 which connects opposing side wall sections 61. The chamber 53 is also defined by a curved end wall 63, which is profiled to match a part of the spherical part of the engagement element 29. The curved end wall 63, side wall sections 61 and base wall 55 intersect at a dished transition surface 65 which is also profiled to match a part of the spherical part of the engagement element 29. Finally, the profile of the chamber 53 includes a bore 67 which extends from the chamber 53 through the lateral shoulder face 69 of the broach 35. The bore 67 facilitates drainage of the chamber 53.

The bridge section 71 which defines the lateral edge 57 of the slot 49, extends laterally to the lateral shoulder face 69. The bridge section 71 not only constrains the inclination of the broach extraction device 1 and tool 9, right-hand illustration R in Figure 3b, but also provides sufficient strength to the broach 35 to resist any deformation of the side sections 73, for instance anteriorly and/or posteriorly, through use of the broach extraction element 1.

This embodiment of the broach extraction device 1 and the modifications made to the broach 35 to accommodate it are beneficial in retaining the teeth on the anterior and posterior faces of the broach 35. They also mean that there is still space available on or near the lateral shoulder face 69 for any desired alpha/numerical markings such as the broach size. The bridge section 71 is beneficial in constraining the broach extraction element 1 and the tool 9 so that they cannot slip out the lateral side of the broach and potentially impact the trochanter or other elements of the patient's anatomy or soft tissue. The maximum lateral inclination of the broach extraction element 1 and the tool 9 is configured so as to prevent any lateral overhang of the broach extraction element 1 and/or tool 9, for instance to give easier access if the patient's trochanter overhangs.

Whilst the embodiments described above feature one engagement element 29 form for the broach extraction device 1 and one slot 49 and chamber 53 configuration of the broach 53, other forms and configurations are possible.

In the following embodiments, the manner of insertion to give the inserted state and the manner of locking to give the locked state are substantially the same and so are not described in detail and reference is made to the embodiment above. Many of the other features from the embodiment[s] above are also common to the following embodiments and also are not described in detail here either, with again attention being drawn to the embodiment[s] above.

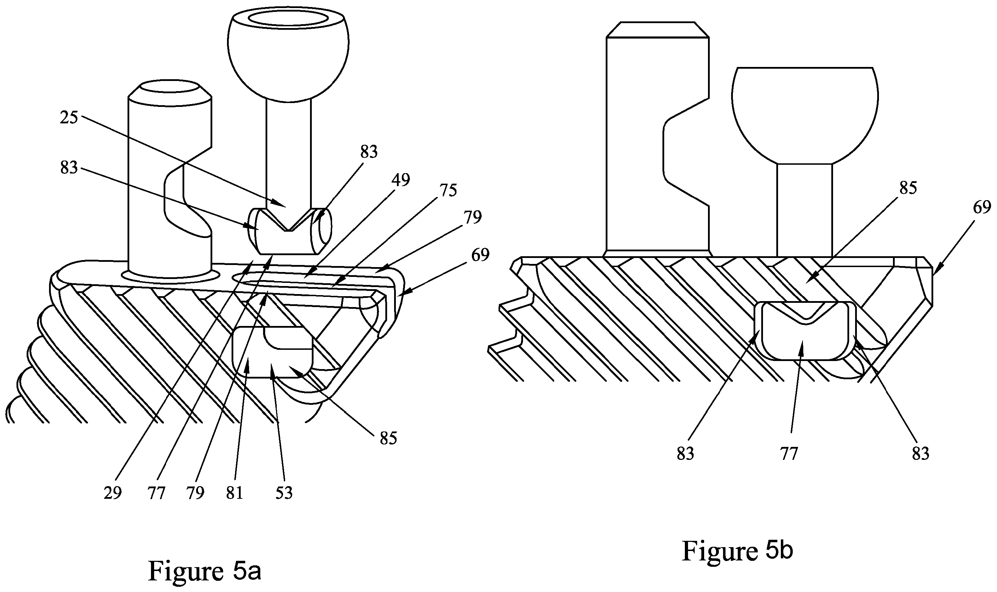

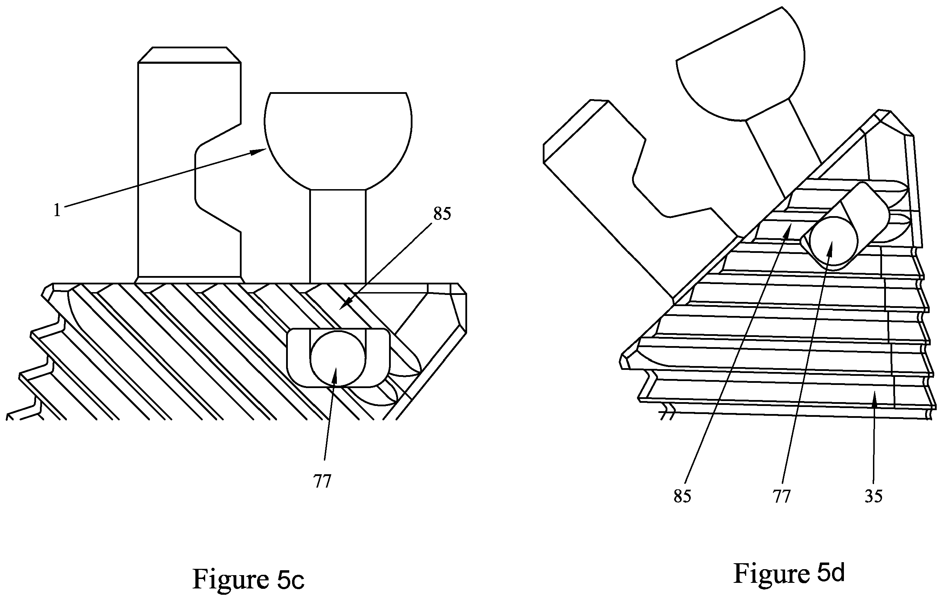

A first alternative embodiment is show in Figures 5a, 5b, 5c and 5d. In this embodiment, the bridge section 71 is not present and the slot 49 is open to the lateral shoulder face 69. The slot extension 75 does not extend distally for the same depth as the chamber 53 and so an inclined face connects the two. In this case, the broach extraction element still includes a neck 25 which leads to the engagement element 29, but in this case the engagement element 29 has a different profile. A right cylindrical part 77 provides the engagement element 29 and the axis of this cylindrical part 77 extends perpendicular to the axis X-X of the broach engagement element 1. In the orientation shown in Figure 5a, the engagement part 29 is able to enter the slot 49 and hence the chamber 53. The posterior and anterior walls 79 of the broach are provided with through apertures 81 which receive the ends 83 of the cylindrical part 77 when that is rotated from the inserted state of Figure 5b to give the locked state of Figure 5c. The apertures 81 assist with drainage from the chamber 53. The cylindrical part 77 abuts the side wall bridges 85 if axial load is applied to pull the broach 35 out. The cylindrical part 77 allows for different orientations of the broach extraction element 1 relative to the broach 35 as seen in Figure 5d.

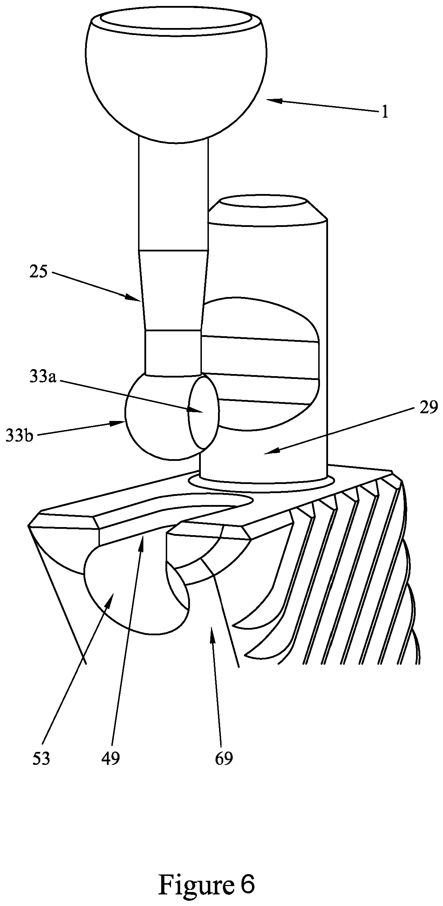

In the second alternative embodiment of Figure 6, the bridge section 71 is again not present and the slot 49 is open to the lateral shoulder face 69, as is the chamber 53. In this embodiment, an engagement element 29 with a configuration similar to the Figure 1 embodiment is used. This includes a part spherical part with flats 33a, 33b on either side.

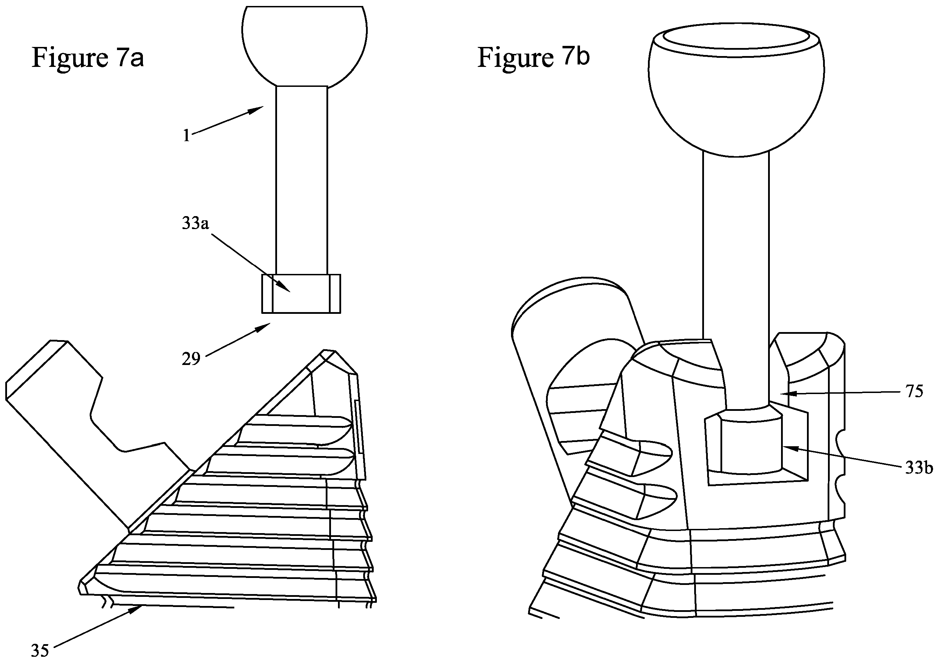

In the fourth alternative embodiment of Figure 7a and 7b, a smaller chamber 53 is provided closer to the lateral shoulder face 69. The chamber 53 and slot 49 are open at the lateral shoulder face 69. Once again, the engagement element 29 can be inserted in one rotational orientation and locked in another orientation where it is too wide to exit through the slot 49. If necessary, the engagement element 29 can be disengaged, or potentially even engaged, without rotation via the opening at the lateral shoulder face 69, accessed via the slot extension 75. In this embodiment, the engagement element 29 has a rectilinear profile, but again uses flats 33a, 33b to truncate two faces of the engagement element 29 and hence provide the different widths.

While the disclosure has been illustrated and described in detail in the drawings and foregoing description, such an illustration and description is to be considered as exemplary and not restrictive in character, it being understood that only illustrative embodiments have been shown and described and that all changes and modifications that come within the scope of the disclosure are desired to be protected.

There are a plurality of advantages of the present disclosure arising from the various features of the apparatus, system, and method described herein. It will be noted that alternative embodiments of the apparatus, system, and method of the present disclosure may not include all of the features described yet still benefit from at least some of the advantages of such features. Those of ordinary skill in the art may readily devise their own implementations of the apparatus, system, and method that incorporate one or more of the features of the present invention and fall within the scope of the present disclosure,

## Claims

1. A surgical instrument extraction device (1), the extraction device (1) comprising:
a body (19);
a first mounting location provided at the proximal end (5) of the body (19);
a second mounting location provided at the distal end (13) of the body (19);
wherein the second mounting location includes an engagement element (29) for the surgical instrument to be extracted in use, the engagement element (29) having a first extent between a first pair of points on opposite sides of the engagement element (29) and having a second extent between a second different pair of points on opposite sides of the engagement element (29), wherein the first extent is greater than the second extent, the engagement element (29) further including one or more non-planar surfaces; **characterised in that**
the one or more non-planar surfaces comprise one or more surface sections which lie on a common sphere.

2. The device (1) of claim 1, wherein the one or more non-planar surface
sections include one or more curved surface sections and/or part-spherical surface sections.

3. The device (1) of claim 1 or claim 2, wherein the non-planar surfaces provide one or more engagement surface sections.

4. The device (1) of claim 3, wherein one or more non-planar engagement
surface sections are provided adjacent a junction between the second mounting location and the body (19).

5. The device (1) of claim 3 or claim 4, wherein one or more non-planar
engagement surface sections are provided distal a junction between the second mounting location and the body (19).

6. The device (1) of any preceding claim, wherein the engagement element (29) comprises one or more planar other surface sections.

7. The device (1) of any preceding claim, wherein the engagement
element (29) comprises two planar other surface sections which are coplanar with one another and/or extend parallel to the longitudinal axis of the extraction device (1).

8. The device (1) of any preceding claim, wherein the engagement
element (29) has the profile of a sphere with one or more spherical caps removed.

9. The device (1) of claim 8, wherein a plane defining a first spherical
cap removed is the plane forming the junction between the engagement element (20) and the elongate portion and/or the plane defining a second spherical cap removed is a first planar other surface and/or the plane defining a third spherical cap removed is a second planar other surface.

10. The device (1) of claim 9, wherein the first planar other surface is
coplanar with the second planar other surface and/or extends parallel to the longitudinal axis of the extraction device (1).

11. The device (1) of any preceding claim, where the first extent is
between a first pair of points on two different non-planar surfaces, for instance on two non-planar surfaces which are in opposition to one another and/or on two non-planar surfaces which are on opposite sides of the engagement element (29) to one another.

12. The device (1) of any preceding claim, wherein the body (19) includes
a body portion and includes an elongate portion, together with a transition portion that connects the body portion to the elongate portion.

13. The device (1) of any preceding claim, wherein the first mounting
location is adapted to connect the extraction device (1) to the distal end of a tool.

14. A kit comprising:
a surgical instrument extraction device (1) as claimed in any preceding claim, and the kit further comprising:
a tool (9) for attachment to the first mounting location of the surgical instrument extraction device (1).

15. The kit of claim 14, wherein the tool further includes a handle and/or
one or more abutment surfaces and/or is adapted for impact by a further tool.

## Patentansprüche

1. Eine Extraktionsvorrichtung (1) für ein chirurgisches Instrument, wobei die Extraktionsvorrichtung (1) Folgendes beinhaltet:
einen Körper (19);
eine erste Befestigungsstelle, die an dem proximalen Ende (5) des Körpers (19) bereitgestellt ist;
eine zweite Befestigungsstelle, die an dem distalen Ende (13) des Körpers (19) bereitgestellt ist;
wobei die zweite Befestigungsstelle ein Eingriffselement (29) für das im Gebrauch zu extrahierende chirurgische Instrument umfasst, wobei das Eingriffselement (29) eine erste Weite zwischen einem ersten Paar Punkte auf gegenüberliegenden Seiten des Eingriffselements (29) aufweist und eine zweite Weite zwischen einem zweiten, unterschiedlichen Paar Punkte auf gegenüberliegenden Seiten des Eingriffselements (29) aufweist, wobei die erste Weite größer als die zweite Weite ist, wobei das Eingriffselement (29) ferner eine oder mehrere nichtplanare Oberflächen umfasst;
**dadurch gekennzeichnet, dass**
die eine oder die mehreren nichtplanaren Oberflächen einen oder mehrere Oberflächenabschnitte beinhalten, die auf einer gemeinsamen Kugel liegen.

2. Vorrichtung (1) gemäß Anspruch 1, wobei der eine oder die mehreren nichtplanaren Oberflächenabschnitte einen oder mehrere gekrümmte Oberflächenabschnitte und/oder teilkugelförmige Oberflächenabschnitte umfassen.

3. Vorrichtung (1) gemäß Anspruch 1 oder Anspruch 2, wobei die nichtplanaren Oberflächen einen oder mehrere Eingriffsoberflächenabschnitte bereitstellen.

4. Vorrichtung (1) gemäß Anspruch 3, wobei ein oder mehrere nichtplanare Eingriffsoberflächenabschnitte angrenzend an eine Verbindungsstelle zwischen der zweiten Befestigungsstelle und dem Körper (19) bereitgestellt sind.

5. Vorrichtung (1) gemäß Anspruch 3 oder Anspruch 4, wobei ein oder mehrere nichtplanare Eingriffsoberflächenabschnitte distal von einer Verbindungsstelle zwischen der zweiten Befestigungsstelle und dem Körper (19) bereitgestellt sind.

6. Vorrichtung (1) gemäß einem der vorhergehenden Ansprüche, wobei das Eingriffselement (29) einen oder mehrere planare andere Oberflächenabschnitte beinhaltet.

7. Vorrichtung (1) gemäß einem der vorhergehenden Ansprüche, wobei das Eingriffselement (29) zwei planare andere Oberflächenabschnitte beinhaltet, die koplanar zueinander sind und/oder sich parallel zu der Längsachse der Extraktionsvorrichtung (1) erstrecken.

8. Vorrichtung (1) gemäß einem der vorhergehenden Ansprüche, wobei das Eingriffselement (29) das Profil einer Kugel aufweist, bei der eine oder mehrere Kugelkappen entfernt wurden.

9. Vorrichtung (1) gemäß Anspruch 8, wobei eine Ebene, die eine erste entfernte Kugelkappe definiert, die Ebene ist, die die Verbindungsstelle zwischen dem Eingriffselement (20) und dem länglichen Anteil bildet, und/oder die Ebene, die eine zweite entfernte Kugelkappe definiert, eine erste planare andere Oberfläche ist, und/oder die Ebene, die eine dritte entfernte Kugelkappe definiert, eine zweite planare andere Oberfläche ist.

10. Vorrichtung (1) gemäß Anspruch 9, wobei die erste planare andere Oberfläche koplanar zu der zweiten planaren anderen Oberfläche ist und/oder sich parallel zu der Längsachse der Extraktionsvorrichtung (1) erstreckt.

11. Vorrichtung (1) gemäß einem der vorhergehenden Ansprüche, wobei die erste Weite zwischen einem ersten Paar Punkte auf zwei unterschiedlichen nichtplanaren Oberflächen, zum Beispiel auf zwei nichtplanaren Oberflächen, die einander gegenüberliegen, und/oder auf zwei nichtplanaren Oberflächen, die sich auf gegenüberliegenden Seiten des Eingriffselements (29) voneinander befinden, besteht.

12. Vorrichtung (1) gemäß einem der vorhergehenden Ansprüche, wobei der Körper (19) einen Körperanteil umfasst und einen länglichen Anteil umfasst, zusammen mit einem Übergangsanteil, der den Körperanteil mit dem länglichen Anteil verbindet.

13. Vorrichtung (1) gemäß einem der vorhergehenden Ansprüche, wobei die erste Befestigungsstelle angepasst ist, um die Extraktionsvorrichtung (1) mit dem distalen Ende eines Werkzeugs zu verbinden.

14. Ein Kit, das Folgendes beinhaltet:
eine Extraktionsvorrichtung (1) für ein chirurgisches Instrument gemäß einem der vorhergehenden Ansprüche, und wobei das Kit ferner Folgendes beinhaltet:
ein Werkzeug (9) zur Anbringung an der ersten Befestigungsstelle der Extraktionsvorrichtung (1) für ein chirurgisches Instrument.

15. Kit gemäß Anspruch 14, wobei das Werkzeug ferner einen Griff und/oder eine oder mehrere Anliegeflächen umfasst und/oder für einen Aufprall durch ein weiteres Werkzeug angepasst ist.

## Revendications

1. Un dispositif d'extraction d'instrument chirurgical (1), le dispositif d'extraction (1) comprenant :
un corps (19) ;
un premier emplacement de montage prévu au niveau de l'extrémité proximale (5) du corps (19) ;
un deuxième emplacement de montage prévu au niveau de l'extrémité distale (13) du corps (19) ;
dans lequel le deuxième emplacement de montage inclut un élément de mise en prise (29) pour que l'instrument chirurgical soit extrait lors de l'utilisation, l'élément de mise en prise (29) ayant une première étendue entre une première paire de points sur des côtés opposés de l'élément de mise en prise (29) et ayant une deuxième étendue entre une deuxième paire différente de points sur des côtés opposés de l'élément de mise en prise (29), la première étendue étant supérieure à la deuxième étendue, l'élément de mise en prise (29) incluant en outre une ou plusieurs surfaces non planes ; **caractérisé en ce que**
les une ou plusieurs surfaces non planes comprennent une ou plusieurs sections de surface qui reposent sur une sphère commune.

2. Le dispositif (1) de la revendication 1, dans lequel les une ou plusieurs sections de surface non planes incluent une ou plusieurs sections de surface incurvées et/ou sections de surface partiellement sphériques.

3. Le dispositif (1) de la revendication 1 ou de la revendication 2, dans lequel les surfaces non planes fournissent une ou plusieurs sections de surface de mise en prise.

4. Le dispositif (1) de la revendication 3, dans lequel une ou plusieurs sections de surface de mise en prise non planes sont prévues de manière adjacente à une jonction entre le deuxième emplacement de montage et le corps (19).

5. Le dispositif (1) de la revendication 3 ou de la revendication 4, dans lequel une ou plusieurs sections de surface de mise en prise non planes sont prévues de manière distale par rapport à une jonction entre le deuxième emplacement de montage et le corps (19).

6. Le dispositif (1) de n'importe quelle revendication précédente, dans lequel l'élément de mise en prise (29) comprend une ou plusieurs autres sections de surface planes.

7. Le dispositif (1) de n'importe quelle revendication précédente, dans lequel l'élément de mise en prise (29) comprend deux autres sections de surface planes qui sont coplanaires l'une avec l'autre et/ou s'étendent parallèlement à l'axe longitudinal du dispositif d'extraction (1).

8. Le dispositif (1) de n'importe quelle revendication précédente, dans lequel l'élément de mise en prise (29) a le profil d'une sphère avec une ou plusieurs coiffes sphériques enlevées.

9. Le dispositif (1) de la revendication 8, dans lequel un plan définissant une première coiffe sphérique enlevée est le plan formant la jonction entre l'élément de mise en prise (20) et la partie allongée et/ou le plan définissant une deuxième coiffe sphérique enlevée est une première autre surface plane et/ou le plan définissant une troisième coiffe sphérique enlevée est une deuxième autre surface plane.

10. Le dispositif (1) de la revendication 9, dans lequel la première autre surface plane est coplanaire avec la deuxième autre surface plane et/ou s'étend parallèlement à l'axe longitudinal du dispositif d'extraction (1).

11. Le dispositif (1) de n'importe quelle revendication précédente, où la première étendue est entre une première paire de points sur deux surfaces non planes différentes, par exemple sur deux surfaces non planes qui sont en opposition l'une par rapport à l'autre et/ou sur deux surfaces non planes qui sont sur des côtés opposés de l'élément de mise en prise (29) l'une par rapport à l'autre.

12. Le dispositif (1) de n'importe quelle revendication précédente, dans lequel le corps (19) inclut une partie corps et inclut une partie allongée, conjointement avec une partie de transition qui raccorde la partie corps à la partie allongée.

13. Le dispositif (1) de n'importe quelle revendication précédente, dans lequel le premier emplacement de montage est conçu pour raccorder le dispositif d'extraction (1) à l'extrémité distale d'un outil.

14. Un kit comprenant :
un dispositif d'extraction d'instrument chirurgical (1) tel que revendiqué dans n'importe quelle revendication précédente, et le kit comprenant en outre :
un outil (9) destiné à être attaché au premier emplacement de montage du dispositif d'extraction d'instrument chirurgical (1).

15. Le kit de la revendication 14, dans lequel l'outil inclut en outre un manche et/ou une ou plusieurs surfaces de butée et/ou est conçu pour un impact par un outil supplémentaire.
